# EUROPEAN PATENT SPECIFICATION

(11) **EP 1 493 737 B1**
(45) Date of publication and mention of the grant of the patent: **06.09.2006**
(21) Application number: 04015753.9
(22) Date of filing: 05.07.2004
(51) Int. Cl.: C07D 309/08, C07D 309/18

(54) **Process for producing a pyran compound**
Verfahren zur Herstellung einer Pyranverbindung
Procédé de préparation d' un compose du pyran

(30) Priority: 04.07.2003 JP 2003270904; 09.09.2003 JP 2003316531; 10.12.2003 JP 2003411432; 19.12.2003 JP 2003422708; 19.12.2003 JP 2003422709
(43) Date of publication of application: 05.01.2005
(73) Proprietor: KAO CORPORATION, Chuo-ku, Tokyo (JP)
(72) Inventor: Umada, Akira, Wakayama-shi Wakayama (JP); Ataka, Yoshiharu, Wakayama-shi Wakayama (JP); Tanaka, Shigeyoshi, Wakayama-shi Wakayama (JP); Naito, Kazuki, Wakayama-shi Wakayama (JP); Mine, Koji, Wakayama-shi Wakayama (JP)
(74) Representative: HOFFMANN EITLE

(56) References cited:
- CH-A- 655 932
- JP-A- 8 127 577
- US-A- 3 681 263

## Description

### Field of the invention

The present invention relates to a process for producing a pyran compound useful as a synthetic intermediate of perfumes, pharmaceutical preparations and agrochemicals.

The present invention relates to a process for producing a mixture of pyran and hydroxypyran.

### Background of the invention

Pyran is an important industrial material of perfumes. For example, 2-phenyl-4-methyldihydropyran can be converted by reductive opening of its pyran ring into 3-methyl-5-phenylpentanol which is particularly important as a perfume (Swiss Patent No. 655932). Further, dihydropyrans in themselves, such as 2-phenyl-4-methyl-3,6-dihydro-2H-pyran, 2-phenyl-4,6-dimethyl-3,6-dihydro-2H-pyran and 2-butyl-4,6-dimethyl-3,6-dihydro-2H-pyran are useful as perfumes (US Patent No. 3681263 and Arm. Khm. Zh., 29(3), pp. 276-277, 1976).

These dihydropyrans can be obtained by reacting an aldehyde such as benzaldehyde with 3-butene-1-ol such as isoprenol in the presence of a catalytic amount of an acid, as described in the above literatures.

JP-B 6-99419 discloses a process for producing dihydropyrans by hetero-Diels-Alder reaction of an aldehyde with a diene in the presence of Lewis acid as a catalyst and a nitro compound as a co-catalyst.

The present inventors found that dihydropyrans can be prepared easily with high productivity and in high yield to solve problems in the processes described above, and they previously filed patent applications therefor (JP-A 10-109980, JP-A 10-338687 and JP-A 11-29564).

JP-A 8-127577 discloses that a pyran compound is obtained by dehydration in the presence of a sulfate.

### Summary of the invention

The present invention relates to a process for producing a mixture of:
a pyran represented by the formula (II): 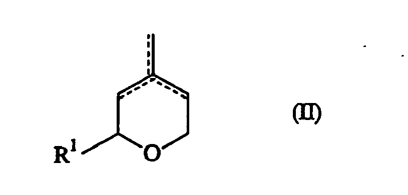 wherein R¹ represents a hydrogen atom, an alkyl or alkenyl group having 1 to 12 carbon atoms, an optionally alkyl-substituted cycloalkyl group having 3 to 12 carbon atoms in total, or an optionally alkyl- or alkoxy-substituted aryl group having 6 to 12 carbon atoms in total, and 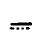 is a single or double bond, and
a hydroxypyran represented by the formula (III): 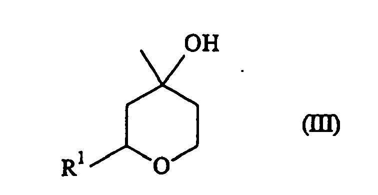 wherein R¹ has the same meaning as defined above,
which comprises reacting an aldehyde represented by the formula (I) (hereinafter, referred to as aldehyde (I)) with isoprenol:

R¹-CHO (I)

wherein R¹ has the same meaning as defined above,
wherein the reaction is initiated in a system where the aldehyde (I)/isoprenol molar ratio is higher than 1.

In addition, the present invention provides a process for producing a pyran compound represented by the above shown formula (II), which comprises the following steps 1 and 2:
step 1: a step of initiating the reaction of an aldehyde represented by the formula (I) with isoprenol in the absence of a solvent, in a system wherein the R¹CHO/isoprenol molar ratio is higher than 1, to give a mixture of a pyran compound represented by the formula (II) and a hydroxypyran compound represented by the formula (III) and
step 2: a step of subjecting the hydroxypyran compound in the mixture obtained in step 1 to dehydration reaction in the presence of an acid to give a pyran compound represented by the formula (II).

### Detailed Description of the invention

In the process described in Swiss Patent No. 655932, there is a problem that the reaction yield of a mixture of pyran and hydroxypyran may be lowered, and when a solvent such as toluene is used in a large amount, productivity is lowered.

In the process described in JP-A 10-109980,JP-A 10-338687 and JP-A 11-29564, the pyran compound can be produced at a high yield, but a halogenated Lewis acid catalyst such as aluminum chloride is used, and therefore a hydrogen halide gas can be generated during the reaction, thus requiring corrosion-resistant production facilities. The diene compound such as isoprene is highly flammable and polymerizable, thus requiring facilities handled at low temperatures. The present invention provides a process for producing a pyran compound efficiently with high productivity without any limitation to the reaction unit.

According to the process of the present invention, a pyran compound useful as a synthetic intermediate of perfumes, pharmaceutical preparations or agrochemicals can be produced efficiently with high productivity without any limitation to the reaction unit.

According to the process of the present invention, a mixture of pyran and hydroxypyran as important industrial starting materials of perfumes can be produced efficiently and economically with simplified production facilities.

In the present invention, the aldehyde (I) is reacted with isoprenol to produce a mixture of the pyran represented by the formula (II) and the hydroxypyran represented by the formula (III). Further, the hydroxypyran compound in the resulting mixture can be subjected to dehydration reaction in the presence of an acid to give the pyran compound represented by the formula (II). Hereinafter, the former and latter are referred to as the first and second steps respectively and described in detail.

In the aldehyde (I) used in the first step of the present invention, R¹ represents a hydrogen atom, an alkyl or alkenyl group having 1 to 12 carbon atoms, an optionally alkyl-substituted cycloalkyl group having 3 to 12 carbon atoms in total, or an optionally alkyl- or alkoxy- substituted aryl group having 6 to 12 carbon atoms in total, preferably an alkyl group having 3 to 12 carbon atoms or an optionally alkyl-substituted aryl group having 6 to 12 carbon atoms in total, more preferably an optionally alkyl-substituted aryl group having 6 to 12 carbon atoms in total, even more preferably a phenyl group or an o-, m- or p-tolyl group. Examples of aldehyde (I) include benzaldehyde, o-, m- or p-tolualdehyde, naphthoaldehyde, butylaldehyde, valeraldehyde, capronaldehyde, heptaldehyde, caprylaldehyde, caprinaldehyde, laurinaldehyde etc., among which benzaldehyde and o-, m- or p-tolualdehyde are preferable, and benzaldehyde is particularly preferable.

The isoprenol used in step 1 is a compound represented by formula (IV) , and can be easily produced from isobutylene and formaldehyde.

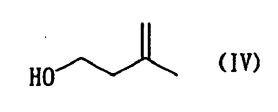

The catalyst used in step 1 includes methanesulfonic acid, p-toluenesulfonic acid, sulfuric acid, hydrochloric acid etc., among which methanesulfonic acid and p-toluenesulfonic acid are preferable. The amount of the catalyst used is usually 0.05 to 5 mol-%, preferably 0.1 to 1 mol-%, relative to aldehyde (I).

From the viewpoint of improving the yield of the objective mixture in the reaction in step 1, the reaction should be initiated in a system wherein the aldehyde (I)/isoprenol molar ratio is higher than 1. The method of initiating the reaction in a system wherein the aldehyde (I) /isoprenol molar ratio is higher than 1 includes a method that involves charging a reactor with the whole of aldehyde (I) and then adding isoprenol to it dropwise to react the aldehyde with isoprenol, a method that involves charging a reactor with a part of aldehyde (I) and then adding a mixed solution of isoprenol and the rest of aldehyde (I) to it dropwise to react the aldehyde with isoprenol, a method that involves adding a mixed solution of aldehyde (I) and isoprenol wherein the aldehyde (I)/isoprenol molar ratio is higher than 1, to a reactor dropwise to react the aldehyde with isoprenol, and a method that involves previously charging a reactor with the whole of a mixture wherein the aldehyde (I)/isoprenol molar ratio is higher than 1 and then initiating the reaction, among which the method that involves charging a reactor with the whole of aldehyde (I) and then adding isoprenol to it dropwise to react the aldehyde with isoprenol is preferable. Preferably, the time for dropwise addition is 1 hour or more from the viewpoint of inhibiting the formation of byproducts, or 12 hours or less from the viewpoint of productivity. Accordingly, the time for dropwise addition is preferably 1 to 12 hours, more preferably 1 to 8 hours.

In the reaction in step 1, the final molar ratio of aldehyde (I) to isoprenol may be selected such that aldehyde (I) is in excess or isoprenol is in excess, but from the viewpoint of improving the yield, the aldehyde (I)/isoprenol ratio (molar ratio) is preferably 1 to 10, more preferably 1 to 3.5.

The ratio of pyran to hydroxypyran formed can also be regulated according to the final molar ratio of aldehyde (I) to isoprenol charged. As the aldehyde (I)/isoprenol ratio (molar ratio) is increased, the ratio of hydroxypyran is increased, while as the aldehyde (I)/isoprenol ratio (molar ratio) is decreased, the ratio of pyran is increased. Accordingly, the final molar ratio of aldehyde (I) to isoprenol charged is preferably 3 or more, more preferably 3 to 6, in order to obtain hydroxypyran selectively in high yield.

To improve the productivity, the reaction in step 1 is conducted in the absence of a solvent.

To improve the reaction rate, the reaction temperature in step 1 is preferably 40°C or more. The reaction temperature is more preferably 40 to 120°C because when the reaction temperature is too high, the decomposition and side reaction of isoprenol proceed easily. The reaction pressure is not particularly limited, but the yield of pyran can be increased by removing formed water from the system. For this purpose, the reaction pressure is preferably 1 to 101.3 kPa, more preferably 1 to 40 kPa. To remove formed water from the system, units such as a packed column and a dehydrating tube may also be used. After isoprenol or a mixed solution of isoprenol and aldehyde (I) was added dropwise, aging may be carried if necessary to increase the degree of conversion. As a general rule, the temperature and pressure during aging may be the same as during dropwise addition. The aging time is not particularly limited, but is preferably about 1 to 8 hours because the decomposition, polymerization etc. of the reaction product proceed when the aging time is too long.

The catalyst used in the second step of the present invention includes acidic compounds such as phosphoric acid, sulfuric acid, methanesulfonic acid and p-toluenesulfonic acid, sulfates such as potassium hydrogen sulfate, sodium hydrogen sulfate, copper sulfate, sodium sulfate, nickel sulfate, magnesium sulfate and zirconium sulfate, alumina etc. Among these compounds, the acidic compounds are preferable, and phosphoric acid, methanesulfonic acid and p-toluenesulfonic acid are more preferable. The catalyst used in step 1, such as methanesulfonic acid or p-toluenesulfonic acid, can be used in step 2 as it is. The amount of the catalyst used is preferably 0.01 to 1% by weight, more preferably 0.02 to 0.5% by weight, relative to the mixture obtained in step 1.

In the dehydration reaction in step 2, the reaction is conducted preferably by feeding the hydroxypyran compound (III) intermittently or continuously during reaction. From the viewpoint of suppressing the polymerization and decomposition of the pyran compound, the reaction is carried out preferably under reduced pressure while the formed pyran compound (II) is distilled away. The reaction conditions are not particularly limited, but distillation is carried out preferably at a reduced pressure of 0.1 to 10 kPa. The reaction temperature is preferably 80 to 200°C, more preferably 100 to 180°C. After the step 2 is finished, the remaining hydroxypyran compound can be recovered for reuse in obtaining a pyran compound.

The process for producing a mixture of pyran and hydroxypyran can be carried out in the same manner as in step 1 in the process for producing a pyran compound which comprises steps 1 and 2.

The catalyst used in the process for producing a mixture of pyran and hydroxypyran includes methanesulfonic acid, p-toluenesulfonic acid, sulfuric acid, hydrochloric acid etc., among which methanesulfonic acid and p-toluenesulfonic acid are preferable. The amount of the catalyst added is preferably 0.05 to 5 mol-%, more preferably 0.1 to 1 mol-%, relative to aldehyde (I).

The process for producing a mixture of pyran and hydroxypyran can be carried out with or without a solvent such as toluene, cyclohexane and dichloromethane, but from the viewpoint of improving productivity, the process is carried out preferably without a solvent.

### Examples

Hereinafter, the present invention is described in more detail by reference to the Examples, but the present invention is not limited by the Examples.

### Example 1

72.3 g (0.681 mol) benzaldehyde and 0.33 g (0.0034 mol) methanesulfonic acid were introduced into a 300 ml four-necked flask and mixed at room temperature. This mixture was heated at a pressure reduced to 4.0 kPa in the container until the temperature in the container reached 60°C. After heating, 64.5 g (0.749 mol) isoprenol was added dropwise thereto over 3 hours under stirring, and thereafter, the mixture was aged for 4 hours.

As a result, a mixed solution containing 44.4 g dihydrophenylpyran (0.255 mol, yield 37.4% relative to the charged benzaldehyde) represented by formula (V) below and 52.8 g hydroxyphenyltetrahydropyran (0.275 mol, yield 40.4% relative to the charged benzaldehyde) represented by formula (VI) below was obtained when the aging was finished. The yield in total of the dihydrophenylpyran and hydroxyphenyltetrahydropyran was 77.8%.

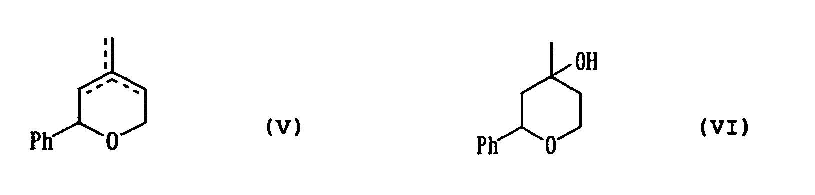

### Example 2

260.1 g (2.451 mol) benzaldehyde and 1.18 g (0.012 mol) methanesulfonic acid were introduced into a 500 ml four-necked flask and mixed at room temperature. This mixture was heated at a pressure reduced to 13.3 kPa in the container until the temperature in the container reached 60°C. After heating, 116.1 g (1.348 mol) isoprenol was added dropwise thereto over 4 hours under stirring, and thereafter, the mixture was aged for 3 hours.

As a result, a mixed solution containing 59.6 g dihydrophenylpyran (0.342 mol, yield 25.4% relative to the charged isoprenol) and 167.7 g hydroxyphenyltetrahydropyran (0.872 mol, yield 64.7% relative to the charged isoprenol) was obtained when the aging was finished. The yield in total of the dihydrophenylpyran and hydroxyphenyltetrahydropyran was 90.1%.

### Example 3

110.0 g (1.036 mol) benzaldehyde and 0.50 g (0.0052 mol) methanesulfonic acid were introduced into a 300 ml four-necked flask and mixed at room temperature. This mixture was heated at a pressure reduced to 4.0 kPa in the container until the temperature in the container reached 60°C. After heating, 26.8 g (0 .311 mol) isoprenol was added dropwise thereto over 3 hours under stirring, and thereafter, the mixture was aged for 4 hours.

As a result, a mixed solution containing 8.69 g dihydrophenylpyran (0.050 mol, yield 16.1% relative to the charged benzaldehyde) and 46.7 g hydroxyphenyltetrahydropyran (0.243 mol, yield 78.1% relative to the charged benzaldehyde) was obtained when the aging was finished. The yield in total of the dihydrophenylpyran and hydroxyphenyltetrahydropyran was 94.2%.

### Example 4

95.2 g (0.897 mol) benzaldehyde and 0.87 g (0.0046 mol) p-toluenesulfonic acid were introduced into a 300 ml four-necked flask and mixed at room temperature. This mixture was heated at a pressure reduced to 1.3 kPa in the container until the temperature in the container reached 60°C. After heating, 42.5 g (0.493 mol) isoprenol was added dropwise thereto over 4 hours under stirring, and thereafter, the mixture was aged for 3 hours.

As a result, a mixed solution containing 35.6 g dihydrophenylpyran (0.204 mol, yield 41.4% relative to the charged isoprenol) and 32.1 g hydroxyphenyltetrahydropyran (0.167 mol, yield 33.8% relative to the charged isoprenol) was obtained when the aging was finished. The yield in total of the dihydrophenylpyran and hydroxyphenyltetrahydropyran was 75.2%.

### Example 5

190.2 g (1.792 mol) benzaldehyde and 0.87 g (0.0091 mol) methanesulfonic acid were introduced into a 300 ml four-necked flask and mixed at room temperature. This mixture was heated at a pressure reduced to 4.0 kPa in the container until the temperature in the container reached 60°C. After heating, 28.6 g (0.332 mol) isoprenol was added dropwise thereto over 3.6 hours under stirring, and thereafter, the mixture was aged for 3.5 hours.

As a result, a mixed solution containing 7.3 g dihydrophenylpyran (0.042 mol, yield 12.7% relative to the charged isoprenol) and 47.9 g hydroxyphenyltetrahydropyran (0.249 mol, yield 75.0% relative to the charged isoprenol) was obtained when the aging was finished. The yield in total of the dihydrophenylpyran and hydroxyphenyltetrahydropyran was 87.7%.

### Example 6

89.44 g (1.038 mol) isovaleraldehyde and 0.51 g (0.0053 mol) methanesulfonic acid were introduced into a 200 ml four-necked flask and mixed at room temperature. This mixture was heated at normal pressures until the temperature in the container reached 60°C. After heating, 26.81 g (0.311 mol) isoprenol was added dropwise thereto over 3 hours under stirring, and thereafter, the mixture was aged for 3 hours.

As a result, a mixed solution containing 15.30 g dihydroisobutylpyran (0.099 mol, yield 31.9% relative to the charged isoprenol) represented by formula (VII) below and 30.81 g hydroxyisobutyltetrahydropyran (0.179 mol, yield 57.5% relative to the charged isoprenol) represented by formula (VIII) below was obtained when the aging was finished. The yield in total of the dihydroisobutylpyran and hydroxyisobutyltetrahydropyran was 89.4%.

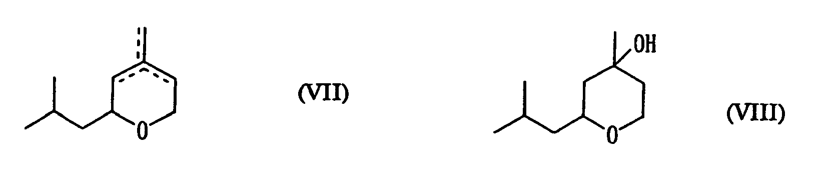

### Example 7

458.5 g (4.32 mol) benzaldehyde and 2.08 g (21.6 mmol) methanesulfonic acid were introduced into a 1 L four-necked flask and heated to 80°C. After heating, 409.3 g (4.75 mol) isoprenol was added dropwise thereto over 3 hours, and the mixture was further aged for 5 hours. As a result of gas chromatographic analysis of the mixture after aging, 233.2 g dihydrophenylpyran (1.34 mol, yield 31.0% relative to the charged benzaldehyde) represented by formula (V) and 439.7 g hydroxyphenyltetrahydropyran (2.29 mol, yield 53.1% relative to the charged benzaldehyde) represented by formula (VI) were obtained. The yield in total of the dihydrophenylpyran and hydroxyphenyltetrahydropyran was 84.0%.

### Example 8

146.2 g mixed solution containing 39.3 g (0.23 mol) dihydrophenylpyran and 73.1 g (0.38 mol) hydroxyphenyltetrahydropyran, produced in the same manner as in Example 7, was used as a dehydration reaction material.

40.2 g of the above material and 0.47 g (4.1 mmol) of 85% phosphoric acid were introduced into a 100 ml four-necked flask and heated to 135°C at a reduced pressure of 0.67 kPa. As the product was distilled away, the rest (106.0 g) of the above material was fed thereto. The mixture was reacted for 8.1 hours to give 89.0 g (0.51 mol, yield 84.3%) dihydrophenylpyran represented by formula (V). The reaction productivity defined as the amount of dihydrophenylpyran (V) formed per L of the reaction container was 890 g/L.

The productivity is defined as the amount of dihydrophenylpyran (V) formed per L of the reaction container.

### Example 9

144.2 g mixed solution containing 61.2 g (0.35 mol) dihydrophenylpyran and 60.5 g (0.31 mol) hydroxyphenyltetrahydropyran, produced in the same manner as in Example 7, was used as a dehydration reaction material.

40.1 g of the above material and 0.24 g (2.1 mmol) of 85% phosphoric acid were introduced into a 100 ml four-necked flask and heated to 140°C at a reduced pressure of 1.33 kPa. As the product was distilled away, the rest (104.1 g) of the above material was fed thereto. The mixture was reacted for 6.2 hours to give 100.9 g (0.58 mol, yield 87.0%) dihydrophenylpyran represented by formula (V). The productivity of dihydrophenylpyran (V) was 1009 g/L.

### Example 10

138.0 g (1.30 mol) benzaldehyde and 0.63 g (6.5 mmol) methanesulfonic acid were introduced into a 300 ml four-necked flask and heated to 60°C. After heating, 123.2 g (1.43 mol) isoprenol was added dropwise thereto over 4.1 hours, and the mixture was further aged for 9 hours. As a result of gas chromatographic analysis of the mixture after the reaction, 71.6 g (0.41 mol) dihydrophenylpyran and 129.5 g (0.67 mol) hydroxyphenyltetrahydropyran had occurred.

Then, the above mixture was distilled at a reduced pressure of 0.67 kPa to give 95.7 g (0.55 mol, yield 42.3%) dihydrophenylpyran represented by formula (V). The productivity of dihydrophenylpyran (V) was 319 g/l.

### Example 11

72.2 g (0.68 mol) benzaldehyde and 0.13 g (6.6 mmol) p-toluenesulfonic acid were introduced into a 200 ml four-necked flask and heated to 80°C. After heating, 123.2 g (1.43 mol) isoprenol was added dropwise thereto over 3.2 hours, and the mixture was further aged for 5 hours. As a result of gas chromatographic analysis of the mixture after the reaction, 70.2 g (0.40 mol) dihydrophenylpyran and 132.4 g (0.69 mol) hydroxyphenyltetrahydropyran had occurred.

Then, the above mixture was distilled at a reduced pressure of 0.67 kPa to give dihydrophenylpyran (V), 57.4 g (0.33 mol, yield 45.8%). The productivity of dihydrophenylpyran (V) was 295 g/l.

### Example 12

138.0 g (1.30 mol) benzaldehyde and 0.63 g (0.7 mmol) methanesulfonic acid were introduced into a 300 ml four-necked flask and heated to 120°C. After heating, 64.5 g (0.75 mol) isoprenol was added dropwise thereto over 2.5 hours, and the mixture was further aged for 4 hours. As a result of gas chromatographic analysis of the mixture after the reaction, 44.2 g (0.25 mol) dihydrophenylpyran and 44.7 g (0.23 mol) hydroxyphenyltetrahydropyran had occurred.

Then, the above mixture was neutralized with NaOH, and 1.4 g (12.1 mmol) of 85% phosphoric acid was added thereto, and the mixture was distilled at a reduced pressure of 0.67 kPa to give dihydrophenylpyran (V), 119.7 g (0.69 mol, yield 53.1%). The productivity of dihydrophenylpyran (V) was 399 g/l.

### Example 13

138.1 g (1.30 mol) benzaldehyde and 0.22 g (1.3 mmol) p-toluenesulfonic acid were introduced into a 300 ml four-necked flask and heated to 60°C. After heating, 123.2 g (1.43 mol) isoprenol was added dropwise thereto over 2.8 hours, and the mixture was further aged for 10 hours. As a result of gas chromatographic analysis of the mixture after the reaction, 62.3 g (0.36 mol) dihydrophenylpyran (V) and 121.3 g (0.63 mol) hydroxyphenyltetrahydropyran (VI) had occurred.

Then, the above mixture was distilled at a reduced pressure of 0.67 kPa to effect the dehydration reaction of hydroxyphenyltetrahydropyran (VI), to give dihydrophenylpyran (V) , 122.8 g (0.70 mol, yield 54.2%). The amount of unreacted hydroxyphenyltetrahydropyran (VI) was 53.4 g (0.28 mol). The productivity of dihydrophenylpyran (V) was 409 g/L.

### Example 14

90.2 g (0.85 mol) benzaldehyde and 0.41 g (4.3 mmol) methanesulfonic acid were introduced into a 200 ml four-necked flask and heated to 60°C. After heating, 80.6 g (0.94 mol) isoprenol was added dropwise thereto over 3.2 hours, and the mixture was further aged for 5 hours. As a result of gas chromatographic analysis of the mixture after the reaction, 45.9 g (0.26 mol) dihydrophenylpyran (V) and 86.5 g (0.45 mol) hydroxyphenyltetrahydropyran (VI) had occurred.

Then, the above mixture was neutralized with NaOH, and after 40.1 g of the mixture was placed in a 100 ml four-necked flask, 0.2 g (1 .7 mmol) of 85% phosphoric acid was added thereto, and the mixture was heated to 130°C at a reduced pressure of 1.33 kPa. As the product was distilled away, the rest (122.0 g) of the mixture was fed thereto. The mixture was subjected to dehydration reaction for 7.3 hours to give dihydrophenylpyran (V), 108.1 g (0.63 mol, yield 73.0%). The amount of unreacted hydroxyphenyltetrahydropyran (VI) was 5.5 g (0.03 mol). The productivity of dihydrophenylpyran (V) was 541 g/L.

### Comparative Example 1

64.5 g (0.749 mol) isoprenol was introduced into a 300 mL four-necked flask, heated and mixed at a pressure reduced to 9.3 kPa in the container until the temperature in the container reached 60°C. After heating, a mixture prepared previously by mixing 72.3 g (0.681 mol) benzaldehyde with 0.32 g (0.0033 mol) methanesulfonic acid under stirring was added dropwise to it over 3 hours, and thereafter, the reaction mixture was aged for 4 hours.

As a result, a mixed solution containing 34.3 g dihydrophenylpyran (0.197 mol, yield 28.9% relative to the charged benzaldehyde) and 20.6 g hydroxyphenyltetrahydropyran (0.107 mol, yield 15.7% relative to the charged benzaldehyde) was obtained when the aging was finished. The yield in total of the dihydrophenylpyran and hydroxyphenyltetrahydropyran was 44.6%.

### Comparative Example 2

91.6 g mixed solution containing 25.5 g (0.15 mol) dihydrophenylpyran and 45.8 g (0.24 mol) hydroxyphenyltetrahydropyran, produced in the same manner as in Example 7, was used as a dehydration reaction material.

40.1 g of the above material and 0.81 g (5.7 mmol) sodium sulfate were introduced into a 100 ml four-necked flask and heated to 135°C at a reduced pressure of 0.67 kPa. As the product was distilled away, the rest (51.1 g) of the above material was fed thereto. The mixture was reacted for 5.2 hours to give 24.6 g (0.14 mol, yield 34.3%) dihydrophenylpyran represented by formula (V).

### Comparative Example 3

128.2 g (0.14 mol) toluene and 0.20 g (2.1 mmol) methanesulfonic acid were introduced into a 300 ml four-necked flask and heated to 110°C. A mixed solution of 42.5 g (0.40 mol) benzaldehyde and 37.9 g (0.44 mol) isoprenol was added dropwise thereto over 2.6 hours under reflux, and further aged for 1 hour. The reaction mixture was neutralized with 48% NaOH, then washed with water and distilled at a reduced pressure of 0.67 kPa to give 55.5 g (0.32 mol, yield 79.6%) dihydrophenylpyran represented by formula (V). The productivity of dihydrophenylpyran represented by formula (V) was 185 g/l.

## Claims

1. A process for producing a mixture of:
a pyran represented by the formula (II): 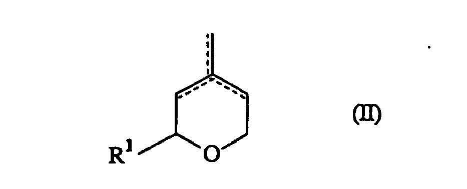 wherein R¹ represents a hydrogen atom, an alkyl or alkenyl group having 1 to 12 carbon atoms, an optionally alkyl-substituted cycloalkyl group having 3 to 12 carbon atoms in total, or an optionally alkyl- or alkoxy-substituted aryl group having 6 to 12 carbon atoms in total, and 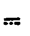 is a single or double bond, and
a hydroxypyran represented by the formula (III): 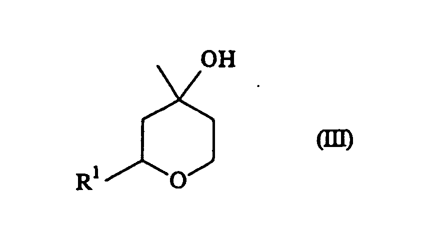 wherein R¹ has the same meaning as defined above,
which comprises the step (called 1^{st} step) of reacting isoprenol with an aldehyde represented by the formula (I) (hereinafter, referred to as aldehyde (I)):
R¹-CHO (I)
wherein R¹ has the same meaning as defined above,
wherein the reaction is initiated in a system where the aldehyde (I)/isoprenol molar ratio is higher than 1.

2. A process for producing a pyran compound represented by the formula (II) from the mixture of the pyran and the hydroxypyran obtained in the 1^{st} step of the process according to claim 1, which comprises the step (called second step) of subjecting the hydroxypyran compound in the mixture to dehydration reaction in the presence of an acid to give a pyran compound represented by the formula (II).

3. The process according to claim 1 or 2, wherein the catalyst used in the first step is at least one member selected from the group consisting of methanesulfonic acid and p-toluenesulfonic acid.

4. The process according to any one of claims 1 to 3, wherein in the first step, the whole of aldehyde is introduced into a reactor, and isoprenol is added thereto dropwise to react the two.

5. The process according to any one of claims 1 to 4, wherein the final molar ratio of aldehyde (1) to isoprenol (aldehyde/isoprenol) charged in the first step is 1 to 10.

6. The process according to any one of claims 1 to 5, wherein the reaction temperature of the 1^{st} step is 40 to 120°C.

7. The process according to any one of claims 1 to 6, wherein R¹ is an optionally alkyl-substituted aryl group having 6 to 12 carbon atoms in total.

8. The process according to any one of claims 1 to 6, wherein R¹ is an alkyl group having 1 to 12 carbon atoms.

9. The process for producing a pyran compound according any of claims 2 to 8, wherein the catalyst used in the second step is phosphoric acid or a sulfonic acid-based compound.

10. The process for producing a pyran compound according to any one of claims 2 to 9, wherein, in the second step, the dehydration reaction is conducted while the hydroxypyran compound (1) is fed intermittently or continuously to the reaction system.

## Patentansprüche

1. Verfahren zur Herstellung einer Mischung aus:
einem Pyran mit der Formel (II): 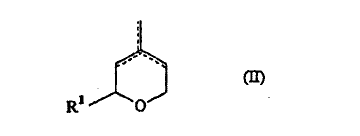 worin R¹ ein Wasserstoffatom, eine Alkyl- oder Alkenylgruppe mit 1 bis 12 Kohlenstoffatomen, eine wahlweise alkylsubstituierte Cycloalkylgruppe mit 3 bis 12 Kohlenstoffatomen insgesamt oder eine wahlweise alkyl- oder alkoxysubstituierte Arylgruppe mit insgesamt 6 bis 12 Kohlenstoffatomen ist und ... eine Einfach- oder Doppelbindung ist, und
einem Hydroxypyran mit der Formel (III): 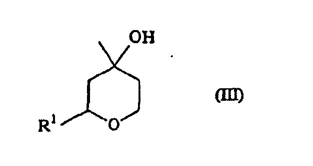 worin R¹ die gleiche Bedeutung, wie oben definiert, aufweist, umfassend den Schritt (erster Schritt genannt) der Reaktion von Isoprenol mit einem Aldehyd mit der Formel (I) (nachfolgend als Aldehyd (I) bezeichnet):
R¹-CHO (I)
worin R¹ die gleiche Bedeutung wie oben aufweist,
worin die Reaktion in einem System initiiert wird, worin das molare Verhältnis von Aldehyd (I)/Isoprenol höher als 1 ist.

2. Verfahren zur Erzeugung einer Pyranverbindung mit der Formel (II) aus der Mischung aus dem Pyran und dem Hydroxypyran, erhalten im ersten Schritt des Verfahrens gemäß Anspruch 1, umfassend den Schritt (zweiter Schritt genannt) der Durchführung einer Dehydratisierungsreaktion mit der Hydroxypyranverbindung in der Mischung in der Gegenwart einer Säure, unter Erhalt einer Pyranverbindung mit der Formel (II) .

3. Verfahren nach Anspruch 1 oder 2, worin der im ersten Schritt verwendete Katalysator zumindest ein Mitglied ist, ausgewählt aus der Gruppe bestehend aus Methansulfonsäure und p-Toluolsulfonsäure.

4. Verfahren nach einem der Ansprüche 1 bis 3, worin im ersten Schritt das gesamte Aldehyd in einen Reaktor eingeführt wird und Isoprenol tropfenweise zur Reaktion der beiden zugegeben wird.

5. Verfahren nach einem der Ansprüche 1 bis 4, worin das endgültige molare Verhältnis von Aldehyd (1) zu Isoprenol (Aldehyd/Isoprenol), die im ersten Schritt zugeführt werden, 1 bis 10 ist.

6. Verfahren nach einem der Ansprüche 1 bis 5, worin die Reaktionstemperatur des ersten Schrittes 40 bis 120°C ist.

7. Verfahren nach einem der Ansprüche 1 bis 6, worin R¹ eine wahlweise alkylsubstituierte Arylgruppe mit insgesamt 6 bis 12 Kohlenstoffatomen ist.

8. Verfahren nach einem der Ansprüche 1 bis 6, worin R¹ eine Alkylgruppe mit 1 bis 12 Kohlenstoffatomen ist.

9. Verfahren zur Erzeugung eine Pyranverbindung nach einem der Ansprüche 2 bis 8, worin der im zweiten Schritt verwendete Katalysator eine Verbindung auf Basis von Phosphorsäure oder Sulfonsäure ist.

10. Verfahren zur Erzeugung einer Pyranverbindung nach einem der Ansprüche 2 bis 9, worin im zweiten Schritt die Dehydratisierungsreaktion durchgeführt wird, während die Hydroxypyranverbindung (1) absatzweise oder kontinuierlich zum Reaktionssystem geführt wird.

## Revendications

1. Procédé pour produire un mélange de :
un pyranne représenté par la formule (II) : 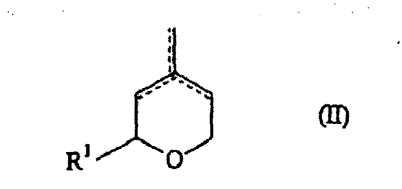 dans laquelle R¹ représente un atome d'hydrogène, un groupe alkyle ou alcényle ayant 1 à 12 atomes de carbone, un groupe cycloalkyle éventuellement alkyl-substitué ayant 3 à 12 atomes de carbone au total, ou un groupe aryle éventuellement alkyl- ou alcoxy-substitué ayant 6 à 12 atomes de carbone au total, et 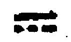 est une liaison simple ou double, et
un hydroxypyranne représenté par la formule (III): 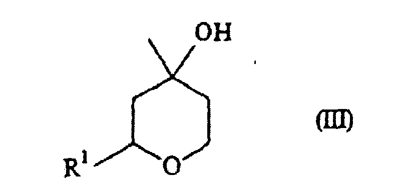
dans laquelle R¹ a la même signification que défini ci-dessus,
qui comprend l'étape (nommée 1ère étape) de faire réagir de l'isoprénol avec un aldéhyde représenté par la formule (I) (ci-après désigné sous le nom de aldéhyde (I)) :
R¹-CHO (I)
dans laquelle R¹ a la même signification que défini ci-dessus,
dans lequel la réaction est lancée dans un système où le rapport molaire aldéhyde (I) / isoprénol est supérieur à 1.

2. Procédé pour produire un composé de pyranne représenté par la formule (II) à partir du mélange de pyranne et d'hydroxypyranne obtenu dans la première étape du procédé selon la revendication 1, qui comprend l'étape (nommée seconde étape) de soumettre le composé d'hydroxypyranne dans le mélange à une réaction de déshydratation en présence d'un acide pour donner un composé de pyranne représenté par la formule (II).

3. Procédé selon la revendication 1 ou 2, dans lequel le catalyseur utilisé dans la première étape est au moins un membre choisi dans le groupe constitué par l'acide méthanesulfonique et l'acide p-toluènesulfonique.

4. Procédé selon l'une quelconque des revendications 1 à 3, dans lequel dans la première étape, l'aldéhyde dans sa totalité est introduit dans un réacteur, et l'isoprénol est ajouté à celui-ci goutte à goutte pour faire réagir les deux.

5. Procédé selon l'une quelconque des revendications 1 à 4, dans lequel le rapport molaire final de l'aldéhyde (1) à l'isoprénol (aldéhyde / isoprénol) chargés dans la première étape est de 1 à 10.

6. Procédé selon l'une quelconque des revendications 1 à 5, dans lequel la température de réaction de la 1ère étape est de 40 à 120°C.

7. Procédé selon l'une quelconque des revendications 1 à 6, dans lequel R¹ est un groupe aryle éventuellement alkyl-substitué ayant 6 à 12 atomes de carbone au total.

8. Procédé selon l'une quelconque des revendications 1 à 6, dans lequel R¹ est un groupe alkyle ayant 1 à 12 atomes de carbone.

9. Procédé pour produire un composé de pyranne selon l'une quelconque des revendications 2 à 8, dans lequel le catalyseur utilisé dans la seconde étape est l'acide phosphorique ou un composé à base d'acide sulfonique.

10. Procédé pour produire un composé de pyranne selon l'une quelconque des revendications 2 à 9, dans lequel, dans la seconde étape, la réaction de déshydratation est effectuée alors que le composé d'hydroxypyranne (1) est chargé de manière intermittente ou de manière continue dans le système réactionnel.
